**Europäisches Patentamt**

**European Patent Office**

**Office européen des brevets**

(11) Publication number : **0 315 681 B1**

# EUROPEAN PATENT SPECIFICATION

(45) Date of publication of patent specification :
**30.09.92 Bulletin 92/40**

(51) Int. Cl.⁵ : **A61K 31/00**

(21) Application number : **88905522.4**

(22) Date of filing : **06.06.88**

(86) International application number :
**PCT/US88/01836**

(87) International publication number :
**WO 88/09603 15.12.88 Gazette 88/27**

(54) **Use of nalmefene for the manufacture of a pharmaceutical composition for central nervous system injury treatment.**

(30) Priority : **05.06.87 US 58340**

(43) Date of publication of application :
**17.05.89 Bulletin 89/20**

(45) Publication of the grant of the patent :
**30.09.92 Bulletin 92/40**

(84) Designated Contracting States :
**AT BE CH DE FR GB IT LI LU NL SE**

(56) References cited :
**Peptides, volume 6, Suppl. 1, 1985, pages 15-17**
**Neurology, volume 35, number 9, 1985, pages 1311-1315**

(56) References cited :
**American Journal of Physiology: Endocrinlogy and Metabolism, volume 16, number 5, November 1987, pages E565-E574**
**Ann. Neurol., volume 17, number 4, 1985, pages 386-390**

(73) Proprietor : **MEDICIS CORPORATION**
**1747 Pennsylvania Avenue N.W.**
**Washington, DC 20006 (US)**

(72) Inventor : **FADEN, Alan, I.**
**406 Wendy Way**
**Mill Valley, CA 94941 (US)**

(74) Representative : **Sternagel, Hans-Günther, Dr. et al**
**Patentanwälte Dr. Michael Hann Dr. H.-G. Sternagel Sander Aue 30**
**W-5060 Bergisch Gladbach 2 (DE)**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue Saint-Denis, 75001 PARIS

## Description

Endogenous opioids may be released following traumatic or ischemic injury of the central nervous system. These opiods may serve as secondary pathophysiologic factors contributing to the neurological disorder which stems from the injury to the central nervous system. Opiate receptor antagonists, such as naloxane, have been used to treat brain or spinal cord injury at dosages in the range of 1 to 10 mg/kg of body weight of the patient.

However, naloxane is not completely selective nor a pure opiate antagonist in all situations. At low dosages, naloxone has considerable selectivity for the mu-opiate receptor. At higher dosages, naloxone acts on other opiate receptors, including the delta and kappa receptors. Further at higher dosages, naloxone may have effects that are not mediated by opiate receptors.

In order to simplify and enhance the safety of central nervous system protocols, opiate receptor antagonists which exhibit a high degree of specificity for or enhanced activity at a specific opiate receptor are being sought. Also, opiate receptor antagonists which act exclusively as such without producing any undesirable side reactions within the body are preferred.

Life Sciences, Vol. 40, pages 1287 to 1292 (1987) discloses the opioid antagonist activities of two bivalent ligands, binaltorphimine and norbinaltorphimine, have been evaluated in smooth muscle preparations and in mice.

Both ligands are highly potent and selective as kappa-opioid receptor antagonists, with relatively feable blocking activity at $\mu$ and $\delta$ opioid receptors.

Likewise the publications Peptides, Vol. 6, Supplement 1, 1985, pages 15 to 17, Neurology, Vol. 35, No. 9, 1985, pages 1311 to 1315 and Annals of Neurology, Vol. 17, No. 4, 1985, pages 386 to 390 do not disclose or suggest any pharmaceutical data of nalmefene but merely are based on studies using WIN 44,441-3.

It is the object of the present invention to provide the use of a specific pharmaceutically active ingredient for the manufacture of an injectable pharmaceutical composition for the treatment of ischemic or traumatic central nervous system injury wherein said active ingredient has an enhanced activity at the kappa-opiate receptor.

This object is attained using nalmefene as a pharmaceutically active ingredient of said pharmaceutical composition.

The present invention provides the use of nalmefene or a salt thereof as opiate-receptor antagonist for the manufacture of an injectable pharmaceutical composition for treatment of ischemic or traumatic central nervous system injury wherein nalmefene has an enhanced activity at the kappa-opiate receptor.

Preferably, nalmefene is present in a concentration of 1 mg/cm³.

Preferably, the composition is based on an isotonic solution comprising water, salt and conventional auxiliaires.

As an opiate-receptor antagonist of the present invention there is contemplated nalmefene or salt thereof having enhanced activity at the kappa-opiate receptor capable of inducing opiate receptor antagonistic activity.

As an effective amount of said opiate-receptor antagonist of the present invention there is contemplated an amount of antagonist which is sufficient to induce Kappa opiate receptor antagonistric activity. An effective amount of the opiate receptor antagonist of the present invention is from 0.01 to 10 mg/kg body weight of the patient daily. A preferred embodiment of the present invention involves an effective amount of the opiate receptor antagonist from 0.1 to 1 mg/kg body weight of the patient daily.

The opiate receptor antagonist of the present invention may be administered parenterally to the patient in any dosage form convenient under the patient's specific circumstances.

As a parenteral dosage form there is contemplated a dosage unit suitable for intravenous administration which comprises (i) an effective amount of an opiate receptor antagonist having enhanced activity at or specificity for the kappa opiate receptor and (ii) a pharmaceutically acceptable solution.

As a pharmaceutically acceptable solution there is contemplated any solution which is safe for injection and which is biologically inert and hence does not interfere with the active ingredient. As such a pharmaceutically acceptable solution may be mentioned an isotonic solution suitable for injection into a patient. The isotonic solution may contain water, salt and conventional ingredients such as glucose.

The present invention provides a method of inducing opiate-receptor antagonistic activity in a patient suffering from ischemic or traumatic central nervous system injury, wherein the opiate receptor antagonist administered to the patient is nalmefene. Nalmefene can be obtained in accordance with the method of Hahn et al. (J. Med. Chem. 18:259-262, 1975).

Further, the present invention provides a method of inducing opiate-receptor antagonistic activity in a patient suffering from ischemic or traumatic central nervous system injury, wherein said opiate-receptor antagonist is administered in a dosage of from 0.1 mg/kg to 10 mg/kg 1-3 times daily. A preferred embodiment of the present invention provides a method of inducing opiate-receptor antagonistic activity in a patient suffering from ischemic or traumatic central nervous system injury, wherein said opiate-receptor antagonist is administered in a dosage of 0.1 mg/kg 1-3 times daily for nalmefene.

The following illustrate the invention.

EXAMPLE 1

Nalmefene is admixed with 10 cc isotonic solution to obtain a final concentration of active ingredient in the solution of 1 mg/ml.

EXAMPLE 2

Induction of opiate receptor antagonistic activity in a patient suffering from traumatic or ischemic central nervous system injury is accomplished through injection of 0.1 mg/kg of the pharmaceutical preparation of Example 1 2 times daily for 1 day.

## Claims

1. Use of nalmefene or a salt thereof as opiate-receptor antagonist for the manufacture of an injectable pharmaceutical composition for treatment of ischemic or traumatic central nervous system injury wherein nalmefene has an enhanced activity at the kappa-opiate receptor.

2. The use of claim 1 wherein nalmefene is present in a concentration of 1 mg/cm$^3$.

3. The use of claim 1 to 2 wherein the composition is based on an isotonic solution comprising water, salt and conventional auxiliaries.

## Patentansprüche

1. Verwendung von Nalmefen oder einem Salz desselben als Opiatrezeptorantagonist zur Herstellung einer injizierbaren pharmazeutischen Zusammensetzung zur Behandlung ischämischer oder traumatischer Verletzung des zentralen Nervensystems, wobei Nalmefen eine erhöhte Aktivität an dem Kappa-Opiatrezeptor aufweist.

2. Verwendung nach Anspruch 1,
   **dadurch gekennzeichnet,**
   daß Nalmefen in einer Konzentration von 1 mg/cm$^3$ vorhanden ist.

3. Verwendung nach Anspruch 1 oder 2,
   **dadurch gekennzeichnet,**
   daß die Zusammensetzung auf einer Wasser, Salz und konventionelle Hilfsstoffe enthaltenden isotonischen Lösung basiert.

## Revendications

1.- Utilisation de nalmefère ou d'un sel de celui-ci comme anti-récepteur d'opiacé pour la fabrication d'un médicament injectable destiné au traitement de lésion ischémique ou traumatique du système nerveux central, utilisation de nalmefère caractérisée en ce que le nalmefère présente une activité antagoniste renforcée au récepteur de kappa-opiacé.

2.- Utilisation de nalmefère selon la revendication 1, caractérisée en ce que le nalmefère est présent à une concentration de 1 mg/cm$^3$.

3.- Utilisation de nalmefère selon l'une quelconque des revendications 1 et 2, caractérisée en ce que la composition est basée sur une solution isotonique comprenant de l'eau, du sel et des produits auxiliaires classiques.